(19) Europäisches Patentamt
European Patent Office
Office européen des brevets

(11) Numéro de publication: **0 278 875**
**B1**

(12) **FASCICULE DE BREVET EUROPEEN**

(45) Date de publication du fascicule du brevet:
09.05.90

(51) Int. Cl.⁵: **C07D 317/64**

(21) Numéro de dépôt: **88420023.9**

(22) Date de dépôt: **27.01.88**

(54) **Procédé de préparation de méthoxy-3 méthylènedioxy-4,5 benzaldehyde.**

(30) Priorité: **28.01.87 FR 8701175**

(43) Date de publication de la demande:
**17.08.88 Bulletin 88/33**

(45) Mention de la délivrance du brevet:
**09.05.90 Bulletin 90/19**

(84) Etats contractants désignés:
**AT BE CH DE ES FR GB GR IT LI LU NL SE**

(56) Documents cités:
**FR-A- 2 130 656**

**YAKUGAKU ZASSHI,
vol. 103, no. 9, 1983, pages 994-996, JP; M. IINUMA et al.:
"Synthesis of new flavones of Bauhinia championii in
Formosa"
CHEMICAL ABSTRACTS,
vol. 85, 1976, pages 491-492, résumé no. 46191w,
Columbus, Ohio, US; & JP-A-76 19 772 (FUJITA
HARUSHIGE et al.) 17-02-1976**

Le dossier contient des informations techniques
présentées postérieurement au dépôt de la demande et
ne figurant pas dans le présent fascicule.

(73) Titulaire: **RHONE-POULENC CHIMIE, 25, quai Paul
Doumer, F-92408 Courbevoie Cédex(FR)**

(72) Inventeur: **Crochemore, Michel, 3, rue des Lilas Domaine
de Gilbertain, F-69630 Chaponost(FR)**

(74) Mandataire: **Vignally, Noel et al, Rhône-Poulenc
Interservices Service Brevets Chimie Centre de
Recherches des Carrières B.P. 62, F-69192 Saint-Fons
Cédex(FR)**

## Description

La présente invention concerne un procédé simplifié et amélioré de préparation de méthoxy-3 méthylènedioxy-4,5 benzaldéhyde à partir de dihydroxy-4,5 méthoxy-3 benzaldéhyde (ou hydroxyvanilline).

Le méthoxy-3 méthylènedioxy-4,5 benzaldéhyde est un composé très important connu notamment comme intermédiaire dans la synthèse de nombreux alcaloïdes, d'intermédiaires pharmaceutiques et de produits pharmaceutiques.

Diverses voies de synthèse du méthoxy-3 méthylènedioxy-4,5 benzaldéhyde ont été proposées.

Parmi les plus récents, on peut citer les modes de préparation suivants.

Un article de Yakagaku Zasshi, volume 103 pages 994–995 (1988) décrit la préparation de méthylènedioxy-3,4 méthoxy-5 benzaldéhyde par réaction du dihydroxy-3,4 méthoxy-5 benzaldéhyde et du dibromométhane dans le diméthylformamide et en présence de fluorure de potassium représentant 5 fois la quantité molaire du dihydroxy-3,4 méthoxy-5 benzaldéhyde. L'utilisation du diméthylformamide et de quantités prohibitives de fluorure de potassium rendant ce procédé peu intéressant industriellement.

Le brevet FR-A 2 130 656 décrit la préparation de méthylènedioxybenzènes à partir de cathécols éventuellement substitués en présence de bases fortes telles que les tris-amino-méthanes ou les bis-amino-alcoxy-méthanes.

Le résumé n° 85-46191w (1976) des Chemicals Abstracts décrit la préparation de méthylènedioxybenzène par réaction de pyrocatéchol et de diiodométhane dans le diméthylformamide en présence d'une quantité très importante de potasse.

Un article du Bulletin of Chemical Society Japan (MATSUMOTO, 1985, 58 (1), pages 346 à 351) décrit un procédé consistant tout d'abord à faire le méthylènedioxy-4,5 méthoxy-3 benzoate de méthyle par réaction du dibromométhane et du dihydroxy-4,5 méthoxy-3 benzoate de méthyle; puis à réduire la fonction ester en groupement $CH_2OH$ par l'hydrure de lithium et d'aluminium; enfin à oxyder cette fonction alcool primaire ainsi créée en fonction aldéhyde. D'après l'article le rendement global de cette synthèse en trois étapes est de 57%. Ce procédé est beaucoup trop compliqué et est trop peu productif pour pouvoir être utilisé industriellement.

Dans un article de Chemische Zeitung (DALLACKER – 1984, 108, (5) pages 186–187) une autre voie d'accès est décrite; elle consiste à partir de la méthylènedioxy-4,5 méthoxy-3 aniline pour former le bromo-5 méthylènedioxy-1,2 méthoxy-3 benzène; le brome est ensuite remplacé par un groupement aldéhyde. Globalement le rendement de cette synthèse est d'environ 37%. Ce procédé, comme le précédent, est compliqué et trop peu productif pour pouvoir être exploité de manière industrielle.

Un article de Journal of Chemical Society Perkin Trans. (1, 1984 (4) pages 709–712 de Mc KIFTRICK et R. STEVENSON) décrit un mode opératoire permettant de former un pont méthylènedioxy sur l'hydroxyvanilline. La réaction entre l'hydroxyvanilline et le dibromométhane en présence de carbonate de potassium s'effectue dans le diméthylsulfoxyde. Comme la récupération du produit final nécessite un traitement à l'eau, cela rend difficile le recyclage du très coûteux solvant. Aucun rendement n'est précisé.

Les procédés de l'art antérieur comportent généralement la création de la fonction aldéhyde en dernière étape ; le procédé, cité précédemment, consistant à créer un pont méthylènedioxy, sur une molécule comportant déjà la fonction aldéhyde, fait appel à un solvant cher et difficile à recycler.

La présente invention se propose de fournir un procédé de synthèse du méthoxy-3 méthylènedioxy-4,5 benzaldéhyde qui soit simple et qui soit facilement utilisable de manière industrielle.

Elle consiste plus précisément en un procédé de préparation du méthoxy-3 méthylènedioxy-4,5 benzaldéhyde par réaction de dihydroxy-4,5 méthoxy-3 benzaldéhyde et de dihalogénométhane, caractérisé en ce que l'on opère :
- en milieu biphasique eau/composé liquide organique et
- à un pH compris entre 7 et 12.

Le dihydroxy-4,5 méthoxy-3 benzaldéhyde est un composé qui est facilement accessible notamment à partir de vanilline, qui est un produit industriel courant.

L'halogénation, notamment la bromation, sélective de la vanilline, en position ortho par rapport au groupement hydroxyle, se fait industriellement. L'hydrolyse de l'halogénovanilline ainsi obtenu est également une réaction industrielle conduisant au dihydroxy-4,5 méthoxy-3 benzaldéhyde (ou hydroxyvanilline).

Le dihalogénométhane qui peut jouer à la fois le rôle de réactif et de phase organique dans le procédé est plus particulièrement choisi parmi les dérivés symétriques ou mixtes chlorés et bromés. On peut ainsi utiliser le dichlorométhane, le dibromométhane, le chlorobromométhane ou leurs mélanges.

En pratique le dichlorométhane, qui est le composé le plus courant et le moins cher, sera utilisé de préférence.

Le pH du milieu est maintenu entre 7 et 12 tout au long de la réaction. Il est donc nécessaire de neutraliser l'acide halohydrique qui se forme. On peut par exemple ajouter en continu une solution aqueuse alcaline. Généralement on utilisera une solution aqueuse d'un hydroxyde ou d'un carbonate de métal alcalin, le plus souvent une solution aqueuse de soude.

Afin d'avoir une vitesse de réaction suffisante tout en évitant au maximum les réactions secondaires, on opère de préférence à un pH situé entre 8 et 10.

Bien que l'on préfère habituellement utiliser le dihalogénométhane comme phase organique dans le procédé de l'invention, on peut également dissocier son rôle d'agent de pontage, entre les deux fonctions hydroxyle du dihydroxy-4,5 méthoxy-3 benzaldéhyde, de celui de solvant organique.

Dans ce cas, on met en oeuvre au moins une quantité stoechiométrique de dihalogénométhane par rapport au dihydroxy-4,5 méthoxy-3 benzaldéhyde et on utilise un solvant organique non miscible à l'eau. Ce solvant organique peut être quelconque dans la mesure où il est inerte vis-à-vis des réactifs.

Ce peut être notamment un hydrocarbure aromatique tel que par exemple le benzène, le toluène, les chlorobenzènes, les xylènes ; un éther arylaliphatique tel que par exemple l'anisole ; un hydrocarbure aliphatique, ou cycloaliphatique tel que par exemple l'hexane ou le cyclohexane ; un éther aliphatique tel que par exemple le dibutyléther.

Le rapport phase aqueuse/phase organique est tel que l'on ait décantation au repos.

La concentration du dihydroxy-4,5 méthoxy-3 benzaldéhyde dans la phase aqueuse n'est pas critique. Elle dépend le plus souvent de l'étape précédente de préparation dudit dihydroxy-4,5 méthoxy-3 benzaldéhyde.

Il est cependant évident qu'il n'est pas économiquement intéressant d'avoir une concentration trop faible, qui procure une productivité insuffisante de l'appareillage.

Généralement la concentration initiale de dihydroxy-4,5 méthoxy-3 benzaldéhyde dans la phase aqueuse est de 5 % à 50 % en poids.

La réaction est avantageusement catalysée par un catalyseur de transfert de phase classique. On peut se reporter par exemple à l'ouvrage "Phase transfer catalysis" de E.V. DEHMLOV (Monograph in Modern Chemistry, vol. 11 - Verlag Chemie).

Généralement on utilise un dérivé (notamment un halogénure) d'ammonium quaternaire qui est un catalyseur classiquement connu pour ce type de réaction. Ce catalyseur est de préférence recyclé.

La température de réaction n'est pas non plus une caractéristique critique du procédé.

En général on opère à une température de 30°C à 150°C. De préférence cette température sera de 50°C à 120°C.

Comme cela a été indiqué précédemment le dihydroxy-4,5 méthoxy-3 benzaldéhyde peut-être préparé par hydrolyse de l'halogéno-5 hydroxy-4 méthoxy-3 benzaldéhyde. Dans ce cas, il n'est pas nécessaire d'isoler le dihydroxy-4,5 méthoxy-3 benzaldéhyde avant de le faire réagir, selon le procédé de l'invention, avec le dihalogénométhane.

On peut utiliser directement la solution aqueuse obtenue lors de l'étape précédente.

Les exemples qui suivent illustrent l'invention.

## EXEMPLE 1

Dans un réacteur en acier inoxydable de 1,5 litre, muni d'une agitation par ancre, d'un système de chauffage et d'un système d'injection de liquide, on charge 600 g (environ 500 cm3) d'une solution aqueuse à pH 9, contenant sous forme de dérivés sodiques :
- 0,844 mole de dihydroxy-4,5 méthoxy-3 benzaldéhyde,
- 0,200 mole de vanilline (impureté provenant des étapes précédentes de préparation du dihydroxy-4,5 méthoxy-3 benzaldéhyde).

On ajoute ensuite 670 g (environ 500 cm3) de dichlorométhane et 80 g de bromure de tétrabutylammonium.

On ferme le réacteur, on purge à l'azote et on chauffe à 100°C sous agitation.

Le pH du milieu réactionnel est maintenu à une valeur de 9 ± 0,5 par injection d'une solution aqueuse de soude à 30 % de concentration en poids.

Après 5 h 30 min de réaction, on refroidit le mélange réactionnel à température ambiante et on le soutire. Le mélange décante ; on sépare la phase organique inférieure et on extrait la phase aqueuse à l'aide de deux fois 250 cm3 de dichlorométhane.

La phase organique et les extraits obtenus sont réunis.

Le dichlorométhane est distillé afin d'être recyclé dans une opération ultérieure.

Le produit brut obtenu est lavé à l'aide de 250 cm3 d'eau afin d'extraire le bromure de tétrabutylammonium, qui sera également recyclé dans un essai ultérieur.

Le produit brut est alors distillé sous pression réduite (environ 65 Pascals).

On obtient 127,5 g d'une fraction distillant à 110-115°C sous la pression indiquée précédemment.

Les spectres de RMN et de Masse sont conformes à la structure du méthylènedioxy-4,5 méthoxy-3 benzaldéhyde.

Le point de fusion du produit obtenu est de 134°C, ce qui correspond au point de fusion du produit de référence.

Le rendement en méthylènedioxy-4,5 méthoxy-3 benzaldéhyde par rapport au dihydroxy-4,5 méthoxy-3 benzaldéhyde engagé est de 83 %.

## EXEMPLE 2

Cet exemple illustre l'enchaînement des réactions de préparation du dihydroxy-4,5 méthoxy-3 benzaldéhyde à partir du bromo-5 hydroxy-4 méthoxy-3 benzaldéhyde (bromovanilline) et de réaction du dihydroxy-4,5 méthoxy-3 benzaldéhyde ainsi obtenu avec le dichlorométhane.

Dans le réacteur utilisé dans l'exemple 1, on charge :
- 800 cm3 d'eau
- 68,8 g de soude en pastilles
- 101 g de bromo-5 hydroxy-4 méthoxy-3 benzaldéhyde
- 1,6 g de cuivre en poudre.

On chauffe pendant 4 heures à 135°C sous agitation, pour réaliser l'hydrolyse et obtenir le dihydroxy-4,5 méthoxy-3 benzaldéhyde.

On refroidit ensuite à 100°C et on amène le pH à une valeur de 8 par addition de 50 cm3 d'acide sulfurique à 50 %.

On charge alors :
- 265 g (environ 200 cm3) de dichlorométhane
- 14 g de bromure de tétrabutylammonium.

On maintient la température à 100°C.

Le pH est maintenu à 8 ± 0,1) par injection d'une solution aqueuse de soude à 30 % en poids.

En fin de réaction et après les traitements décrits dans l'exemple 1, on sépare une phase aqueuse et une phase organique.

Par chromatographie liquide haute pression (CLHP), on dose les deux phases.

Les résultats sont les suivants :
- taux de transformation du bromo-5 hydroxy-4 méthoxy-3 benzaldéhyde : 100 % ;
- rendement en méthylènedioxy-4,5 méthoxy-3 benzaldéhyde : 41 % ;
- rendement en vanilline (dosée dans la phase aqueuse) : 9 % ;
- rendement en dihydroxy-4,5 méthoxy-3 benzaldéhyde (dosé dans la phase aqueuse) : 34 %.

On ne constate pas de produit de réaction entre les groupements OH de deux molécules et le dichlorométhane.

### EXEMPLE 3

Dans un ballon tricol en verre, muni d'une agitation magnétique, d'un réfrigérant, d'une électrode pour mesurer le pH du milieu réactionnel, d'un système d'introduction de liquide et d'un thermomètre, on charge :
- 250 g (100 cm3) de dibromométhane
- 3,2 g de bromure de tétrabutylammonium
- 17,7 g de dihydroxy-4,5 méthoxy-3 benzaldéhyde en solution dans 250 g d'eau.

La solution aqueuse de dihydroxy-4,5 méthoxy-3 benzaldéhyde provient d'une synthèse enchaînée (connue) en plusieurs phases :
- transformation du méthoxy-2 phénol en di(hydroxyméthyl)-2,4 méthoxy-5 phénol ;
- oxydation du di(hydroxyméthyl)-2,4 méthoxy-5 phénol en diformyl-2,4 méthoxy-5 phénol ;
- réaction de Dakin sur le groupement aldéhyde situé en ortho de l'OH pour former le dihydroxy-4,5 méthoxy-3 benzaldéhyde.

On chauffe sous agitation pendant 2 h 30 min à 80°C, tout en maintenant le pH à environ 9 par addition d'une solution aqueuse de soude.

Après refroidissement et traitement habituel, on dose par CLHP la phase organique :
- taux de transformation du dihydroxy-4,5 méthoxy--3 benzaldéhyde : 100 % ;
- rendement en méthylènedioxy-4,5 méthoxy-3 benzaldéhyde : 100%.

### Revendications

1. Procédé de préparation de méthoxy-3 méthylènedioxy-4,5 benzaldéhyde par réaction de dihydroxy-4,5 méthoxy-3 benzaldéhyde et de dihalogénométhane, caractérisé en ce que l'on opère en milieu biphasique eau/composé liquide organique, à un pH compris entre 7 et 12 et en présence d'un catalyseur de transfert de phase.

2. Procédé selon la revendication 1, caractérisé en ce que le dihalogénométhane est choisi parmi le dichlorométhane, le dibromométhane, le bromochlorométhane et leurs mélanges.

3. Procédé selon l'une des revendications 1 et 2, caractérisé en ce que le dihalogénométhane constitue à la fois le réactif et la phase organique.

4. Procédé selon l'une des revendications 1 et 2, caractérisé en ce que la phase organique comprend un solvant non miscible à l'eau et inerte vis-à-vis des réactifs.

5. Procédé selon la revendication 4, caractérisé en ce que le solvant organique est un hydrocarbure aromatique, un éther arylaliphatique, un hydrocarbure aliphatique ou cycloaliphatique ou un éther aliphatique.

6. Procédé selon l'une des revendications 1 à 5, caractérisé en ce que le catalyseur de transfert de phase est un dérivé d'ammonium quaternaire.

7. Procédé selon l'une des revendications 1 à 6, caractérisé en ce que le pH est maintenu pendant la réaction entre 8 et 10.

8. Procédé selon l'une des revendications 1 à 7, caractérisé en ce que le pH est maintenu aux valeurs souhaitées par addition d'une solution aqueuse alcaline.

9. Procédé selon l'une des revendications 1 à 8, caractérisé en ce que l'on opère à une température de 30°C à 150°C, et de préférence de 50°C à 120°C.

10. Procédé selon l'une des revendications 1 à 9, caractérisé en ce que la concentration initiale de dihydroxy-4,5 méthoxy-3 benzaldéhyde dans la phase aqueuse est de 5 % à 50 % en poids.

### Patentansprüche

1. Verfahren zur Herstellung von 3-Methoxy-4,5-methylendioxy-benzaldehyd durch Reaktion von 4,5-Dihydroxy-3-methoxy-benzaldehyd und Dihalogenmethan, dadurch gekennzeichnet, daß man im Zwei-Phasen-Milieu Wasser/organische, flüssige Verbindung bei einem pH zwischen 7 und 12 und in Gegenwart eines Phasenübergangskatalysators arbeitet.

2. Verfahren gemäß Anspruch 1, dadurch gekennzeichnet, daß das Dihalogenmethan unter Dichlormethan, Dibrommethan, Bromchlormethan und deren Gemischen ausgewählt ist.

3. Verfahren gemäß einem der Ansprüche 1 und 2, dadurch gekennzeichnet, daß das Dihalogenmethan gleichzeitig den Reaktionsteilnehmer und die organische Phase darstellt.

4. Verfahren gemäß einem der Ansprüche 1 und 2, dadurch gekennzeichnet, daß die organische Phase ein mit Wasser nicht mischbares und gegenüber den Reaktionsteilnehmern inertes Lösungsmittel umfaßt.

5. Verfahren gemäß Anspruch 4, dadurch gekennzeichnet, daß das organische Lösungsmittel ein aromatischer Kohlenwasserstoff, ein arylaliphatischer Ether, ein aliphatischer oder cycloaliphatischer Kohlenwasserstoff oder ein aliphatischer Ether ist.

6. Verfahren gemäß einem der Ansprüche 1 bis 5, dadurch gekennzeichnet, daß der Phasenübergangskatalysator ein quaternäres Ammoniumderivat ist.

7. Verfahren gemäß einem der Ansprüche 1 bis 6, dadurch gekennzeichnet, daß das pH während der Reaktion zwischen 8 und 10 gehalten wird.

8. Verfahren gemäß einem der Ansprüche 1 bis 7, dadurch gekennzeichnet, daß das pH durch Zugabe einer wäßrigen Alkalilösung bei den gewünschten Werten gehalten wird.

9. Verfahren gemäß einem der Ansprüche 1 bis 8, dadurch gekennzeichnet, daß man bei einer Temperatur von 30 bis 150°C und vorzugsweise von 50 bis 120°C arbeitet.

10. Verfahren gemäß einem der Ansprüche 1 bis 9, dadurch gekennzeichnet, daß die Anfangskonzentration an 4,5-Dihydroxy-3-methoxy-benzaldehyd in der wäßrigen Phase von 5 bis 50 Gew.-% beträgt.

## Claims

1. Process for the preparation of 3-methoxy-4,5-methylenedioxybenzaldehyde by reacting 4,5-dihydroxy-3-methoxybenzaldehyde and dihalomethane, characterized in that the reaction is carried out in a two-phase medium consisting of water and an organic liquid compound, at a PH of between 7 and 12 and in the presence of a phase transfer catalyst.

2. Process according to Claim 1, characterized in that the dihalomethane is chosen from amongst dichloromethane, dibromomethane, bromochloromethane and their mixtures.

3. Process according to one of Claims 1 and 2, characterized in that the dihalomethane forms both the reagent and the organic phase.

4. Process according to one of Claims 1 and 2, characterized in that the organic phase comprises a solvent which is immiscible in water and inert towards the reagents.

5. Process according to Claim 4, characterized in that the organic solvent is an aromatic hydrocarbon, an arylaliphatic ether, an aliphatic or cycloaliphatic hydrocarbon or an aliphatic ether.

6. Process according to one of Claims 1 to 5, characterized in that the phase transfer catalyst is a quaternary ammonium derivative.

7. Process according to one of Claims 1 to 6, characterized in that during the reaction period, the pH is maintained between 8 and 10.

8. Process according to one of Claims 1 to 7, characterized in that the pH is maintained at the desired values by adding an aqueous alkaline solution.

9. Process according to one of Claims 1 to 8, characterized in that the reaction is carried out at a temperature from 30°C to 150°C, and preferably from 50°C to 120°C.

10. Process according to one of Claims 1 to 9, characterized in that the initial concentration of 4,5-di-hydroxy-3-methoxybenzaldehyde in the aqueous phase is from 5 to 50% by weight.